# EUROPÄISCHE PATENTANMELDUNG

(11) **EP 2 522 309 A1**
(43) Veröffentlichungstag der Anmeldung: **14.11.2012**
(21) Anmeldenummer: 12164374.6
(22) Anmeldetag: 17.04.2012
(51) Int. Cl.: A61F 2/24

(54) **Ventil für eine Herzklappenprothese**

(30) Priorität: 10.05.2011 US 201161484243 P
(71) Anmelder: Biotronik AG, 8180 Bülach (CH)
(72) Erfinder: Fargahi, Amir, 8180 Bülach (CH); Wesselmann, Matthias, 8455 Rüdlingen (CH); Bachmann, Patrice, 8408 Winterthur (CH); Schwitzer, Alwin, 8180 Bülach (CH); Quint, Bodo, 8154 Oberglatt (CH)
(74) Vertreter: Lindner-Vogt, Karin L.

(57) **Zusammenfassung**

Die vorliegende Erfindung betrifft ein Ventil für eine Herzklappenprothese (20) mit einer Ventilmembran (10) aus wenigstens einem Spiralband, das im geschlossenen Zustand der Ventilmembran (10) die Form einer archimedischen Spirale einnimmt, wobei sich die äußeren Randbereiche (14) des Spiralbandes (12) mit einen inneren Randbereich (14) des Spiralbandes (12) einer vorhergehenden Windung der Spirale überlappen.

## Beschreibung

Die Erfindung betrifft ein Ventil für eine Herzklappenprothese.

Die Herzscheidewand trennt das menschliche Herz in zwei Hälften und zwar in eine rechte Herzkammer und einen rechten Vorhof sowie in eine linke Herzkammer und einen linken Vorhof. Zwischen den Herzkammer und Vorhöfen befinden sich vier Herzklappen. Das sauerstoffarme, aber kohlendioxidreiche Blut fließt zunächst durch die Trikuspidalklappe in den rechten Vorhof und von dort aus in die rechte Herzkammer. Die Trikuspidalklappe ist eine dreizipflige Klappe und wird auch als Segelklappe bezeichnet. Von der rechten Kammer gelangt das Blut durch die Pulmonalklappe in die beiden Lungenhälften, wo das Blut wieder mit Sauerstoff angereichert wird. Die Pulmonalklappe ist eine sogenannte Taschenklappe. Das mit Sauerstoff angereicherte Blut gelangt nun aus der Lunge in den linken Vorhof und wird durch die Mitralklappe, die die Form einer zweizipfligen Segelklappe besitzt, in die linke Kammer gepumpt. Schließlich gelangt das Blut von der linken Herzkammer aus durch die Aortenklappe in den großen Blutkreislauf. Die Aortenklappe ist wie die Pulmonalklappe eine Taschenklappe.

Bestehen bei einem Patienten Herzklappenfehler, so ist davon auszugehen, dass sich im Laufe der Zeit die Funktion dieser Herzklappen ständig verschlechtern kann. Der Ersatz von nicht mehr funktionsfähigen Herzklappen durch Herzklappenprothesen ist nach der koronaren Bypassoperation mittlerweile die am häufigsten durchgeführte Operation am menschlichen Herzen. Dabei werden zwei verschiedene Typen von Herzklappenprothesen eingesetzt, nämlich mechanische und biologische Herzklappenprothesen.

Als biologische Herzklappen werden Prothesen bezeichnet, die aus biologischem Material, insbesondere aus den Aortenklappenflügeln von Schweinen oder dem Herzbeutel von Rindem zusammengesetzt werden. Das Gewebe wird zur Fixierung in der Regel chemisch behandelt und auf einem Kunststoff- oder Metallgerüst zur späteren Fixierung im Herzen befestigt.

Biologische Herzklappenprothesen im eingepflanzten Zustand sind allerdings nur begrenzt haltbar, weil die Verkalkung die Klappen zunehmend beeinflusst. Es ist von einer mittleren Lebensdauer von etwa 10 bis 12 Jahren auszugehen. Besonders ausgeprägt ist die schnelle Verkalkung der biologischen Herzklappen jedoch bei jüngeren Patienten. Daher wird die Implantation von biologischen Herzklappen erst im fortgeschrittenen Alter vorgenommen, um ein zweites Auswechseln der Klappe nicht mehr vornehmen zu müssen. Ein mechanischer Herzklappenersatz bedarf einer lebenslangen gerinnungshemmenden Therapie. Allerdings besitzen mechanische Herzklappen gegenüber den biologischen Herzklappen den Vorteil der längeren Haltbarkeit.

Insofern gewinnen künstliche Herzklappen zunehmend an Bedeutung. Ein idealer Herzklappenersatz sollte dabei eine unbegrenzte Haltbarkeit besitzen, ungehinderte Blutflussbedingungen im Gefäß ermöglichen, keine herzklappenbedingte Komplikationen wie erhöhte Thrombogenität oder Endokarditis-Anfälligkeit bedingen, keine prothesenimmanente Risiken wie klappenbedingte Defekte aufweisen, eine einfache Implantation ermöglichen und geräuscharm sein.

Mittlerweile sind Herzklappen entwickelt worden, die eine im Labor nachgewiesene Lebensdauer von 150 Jahren haben. Die hämodynamischen Verhältnisse sind annähernd vergleichbar mit denen natürlicher Herzklappen. Die klappenbedingten Komplikationen wie Gerinnselbildung sind nahezu verschwunden, prothesenbedingte Komplikationen aufgrund einer hervorragenden Weiterentwicklung praktisch nicht mehr aufgetreten. Die mechanischen Herzklappen sind relativ einfach einzupflanzen und das Klappengeräusch der mechanischen Herzklappe wird gut vom Patienten toleriert, da diese - zumindest für die Umwelt - relativ geräuscharm sind.

Viele der derzeit zugelassenen künstlichen Herzklappen können jedoch ausschließlich durch Operation am offenen Herzen implantiert werden, was den Einsatz dieser Prothesen deutlich limitiert, da bei etwas mehr als 1/3 aller Patienten ein hohes Operationsrisiko besteht oder eine solche Operation nicht mehr möglich ist. Aus diesem Grunde sind mittlerweile minimal-invasive Techniken und Herzklappenprothesen entwickelt wurden, bei denen die neue Herzklappe mit Hilfe eines Kathetersystems an den Implantationsort verbracht und verankert wird (zum Beispiel PAVR *percutaneous aortic valve replacement*). Die Verankerung in der Gefäßwand erfolgt in der Regel über ein metallisches Geflecht, dessen Aufbau und Materialwahl dem eines Stents angelehnt ist. Das Geflecht kann selbstexpandierend sein oder wird mittels eines Ballonkatheters aufgeweitet.

Künstliche mechanische Herzklappen, die in Kathetersystemen Verwendung finden sollen, müssen ein für diese Technik ausgelegtes Ventil besitzen. Hier besteht nach wie vor ein hoher Bedarf an Lösungsansätzen.

Aufgabe der vorliegenden Erfindung ist es, einen oder mehrere Nachteile des Standes der Technik zu vermindern oder zu vermeiden. Insbesondere ist es eine Aufgabe der vorliegenden Erfindung ein für die Zwecke der katheterbasierten Implantation optimiertes Ventil für Herzklappenprothese bereitzustellen.

Die vorliegende Erfindung löst diese Aufgabe durch Bereitstellung eines Ventils für eine Herzklappenprothese mit einer Ventilmembran aus wenigstens einem Spiralband, das im geschlossenen Zustand der Ventilmembran die Form einer archimedischen Spirale einnimmt, wobei sich die äußeren Randbereiche des Spiralbandes mit einen inneren Randbereich des Spiralbandes einer vorhergehenden Windung der Spirale überlappen.

Der Erfindung liegt der Gedanke zugrunde, dass sich eine den besonderen Erfordernissen angepasste Ventilmembran für eine Herzklappenprothese, die für den katheterbasierten minimal-invasiven Eingriff ausgelegt ist, durch eine völlig neue Formgebung der Ventilmembran realisieren lässt. Ein Spiralband wird dabei so geformt, dass sich insgesamt eine Kontur einer archimedischen Spirale im geschlossenen Zustand der Ventilmembran ergibt. Dabei überlappen die Ränder des Spiralbandes in Blutstromrichtung derart, dass die inneren Windungen der Spirale jeweils auf einer vorhergehenden Windung zum Liegen kommen. Dadurch ist sichergestellt, dass sich das Ventil nur in Blutstromrichtung öffnen kann.

Im geöffneten Zustand nimmt die Ventilmembran dagegen die Form einer konischen Spirale ein (oder auch kegelförmige Raumspirale). Das Blut kann dann durch die freigegebene Öffnung strömen. Die erfindungsgemäß vorgeschlagene konstruktive Lösung zeichnet sich unter anderem dadurch aus, dass Klappgeräusche verringert oder vermieden werden können. Weiterhin hat sie gegenüber den bisher bekannten Formen den Vorteil, dass sie sich radial der Größe der Gefäße anpassen kann. Dies ist bei Kindern, die sich in der Wachstumsphase befinden, besonders geeignet. Zudem ist die Entfernung/Explantation dieser Herzklappenprothese vereinfacht.

Die Ventilmembran ist an einem umlaufenden Ventilring fixiert, der aus demselben Material wie die Membran bestehen kann. An diesen Ventilring schließt sich in herkömmlicher Weise ein zur Verankerung im Herzen geeignetes Geflecht an.

Das Spiralband besteht vorzugsweise aus metallischen Werkstoffen, beispielsweise Nitinol. Die Oberfläche des Spiralbands kann zudem mit Keramik (zum Beispiel A-SiC), Kunststoff oder einem Wirkstoff beschichtet werden. Zur Vereinfachung der Explantation der Herzklappenprothese kann sie einen röntgensichtbaren Marker aufweisen.

Das Material kann ferner eine Beschichtung zur Verbesserung der Biokompatibilität aufweisen. Die Beschichtung enthält oder besteht aus einem biokompatiblen, anorganischen Werkstoff. Ein biokompatibler, anorganischer Werkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes, der bei bestimmungsgemäßer Verwendung mit der Körperumgebung in Kontakt steht, ist dessen Biokompatibilität (Körperverträglichkeit). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Bevorzugt werden biokompatible Werkstoffe für die Beschichtung verwendet, die im Wesentlichen bioinert sind. Unter bioinerten Werkstoffen werden solche Werkstoffe verstanden, die nach der Implantation über die geplante Standzeit der Herzklappenprothese im Wesentlichen intakt bleiben und keine signifikante Biokorrosion zeigen. Als Prüfmedium zur Testung des Korrosionsverhaltens eines in Frage kommenden Werkstoffs dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe des zu untersuchenden Werkstoffs wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Tagen bis zu mehreren Monaten oder Jahren werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen. Ein Werkstoff gilt insbesondere dann als bioinert, wenn der Werkstoff in oben genanntem Test nach Ablauf von 12 Monaten zu weniger als 10% korrodiert ist.

Weiterhin weist das Spiralband vorzugweise eine Dicke im Bereich von 500 bis 1.000 Mikrometer auf.

Vorzugsweise besitzt die Spirale zwischen 2 bis 12 Windungen.

Nach einer weiteren bevorzugten Ausführungsform ist ein inneres Ende des Spiralbandes als kreisförmige Scheibe ausgebildet.

Ein weiterer Aspekt der Erfindung liegt in der Bereitstellung einer Herzklappenprothese mit einem Ventil gemäß vorgenannter Ausführung.

Die Erfindung wird nachfolgend anhand von Ausführungsbeispielen und den dazugehörigen Figuren näher erläutert. Es zeigen:
- Fig. 1A und 1B: zeigen eine perspektivische Draufsicht sowie eine Seitenansicht auf eine Ventilmembran im geschlossenen Zustand des Ventils.
- Fig. 2A und 2B: zeigen dieselbe Ventilmembran aus den Figuren 1A und 1B in perspektivischer Ansicht und Seitenansicht, jedoch im geöffneten Zustand.
- Fig. 3: zeigt eine erste Ausführungsform einer künstlichen Herzklappenprothese im geschlossenen und geöffneten Zustand.
- Fig. 4: zeigt eine zweite Ausführungsform einer künstlichen Herzklappenprothese im geschlossenen und geöffneten Zustand.
- Fig. 5: zeigt eine Ausführungsform der Ventilmembran mit 2 Spiralbändern.

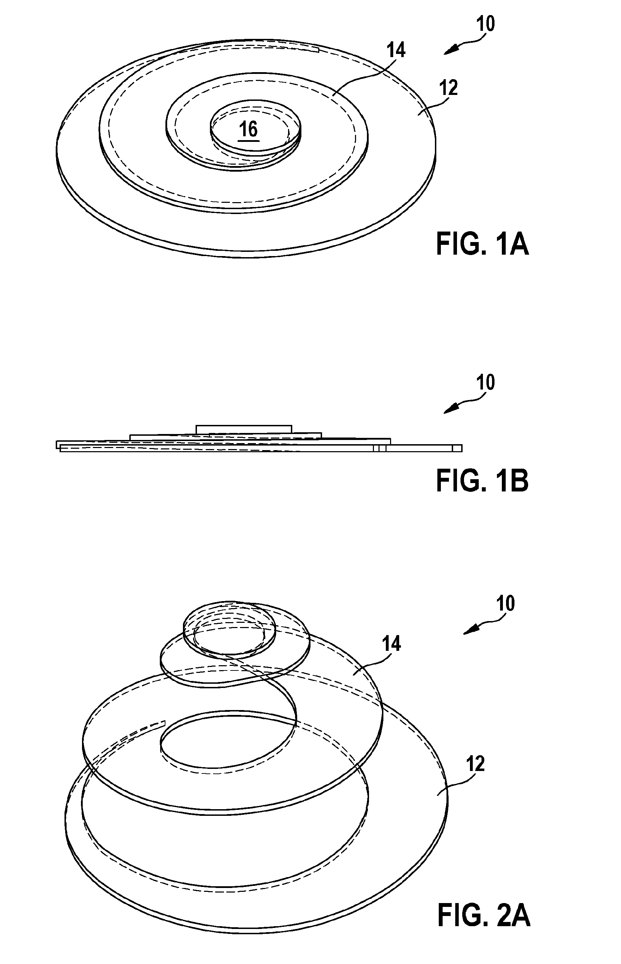

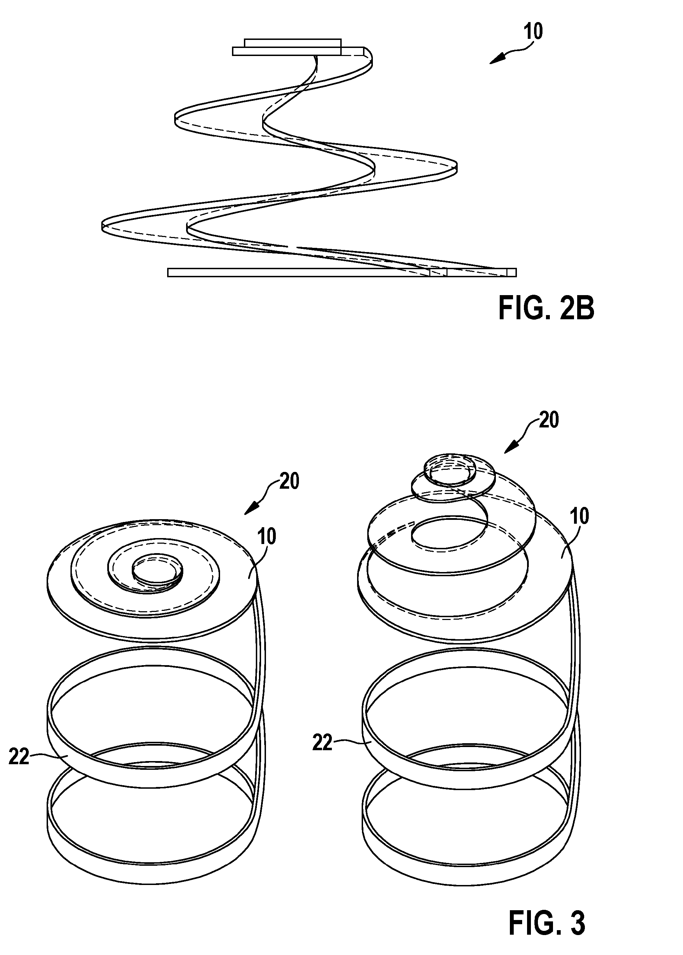

Die Figuren 1A bis 2B zeigen - jeweils in verschiedener perspektivischer Ansicht - eine Ventilmembran 10, die zum Einsatz in einem Ventil für eine Herzklappenprothese ausgelegt ist. Die Ventilmembran 10 ist in den Figuren 1A und 1B im geschlossenen Zustand und in den dazugehörigen Figuren 2A und 2B in gleicher perspektivischer Ansicht im geöffneten Zustand dargestellt. Wie ersichtlich, nimmt die Ventilmembran 10 im geschlossenen Zustand die Form einer archimedischen Spirale ein, deren Grundfläche völlig geschlossen ist. Zu diesem Zwecke ist ein Spiralband 12 derart schneckenförmig übereinander gelegt, dass sich in Laufrichtung die Randbereiche 14 des Spiralbandes 12 mit zunehmender Windung überlappen. Zentral mündet das Spiralband 12 gemäß dem dargestellten Ausführungsbeispiel in einem scheibenförmigen Ende 16.

Die Breite des Spiralbands kann von Außen nach Innen kontinuierlich abnehmen (zum Beispiel von 2.2mm Außen auf 1.2mm Innen). Die Bandstreifen der Spirale überlappen sich. Die Überlappung kann zum Beispiel von 0.2 bis 0.5mm betragen.

Das Spiralband 12 kann beispielsweise aus Nitinol geformt werden und weist eine Dicke von 500 bis 1.000 Mikrometer auf. Die dargestellte Ausführungsform besitzt 3 Windungen. Im geöffneten Zustand nimmt die Spiralmembran 10, wie in den Figuren 2A und 2B dargestellt, die Kontur einer kegelförmigen Raumspirale (konische Spirale) ein, wenn sie rückseitig vom Blutstrom beaufschlagt wird.

Der Figur 3 ist eine Herzklappenprothese 20 im geöffneten und geschlossenen Zustand zu entnehmen, die eine Ventilmembran 10 entsprechend der Figuren 1A bis 2B aufweist. Die Herzklappenprothese 20 wird mit Hilfe ringförmiger Fixierelemente 22 an der vorgesehenen Stelle im Herzen verankert.

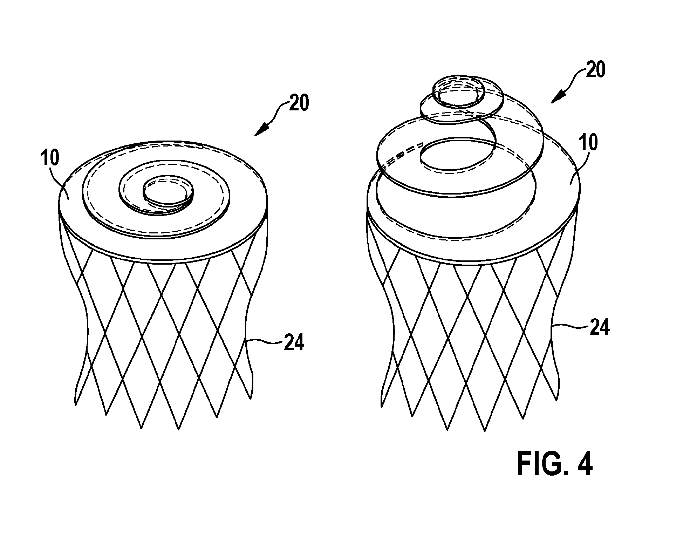

Auch die Herzklappenprothese 20 aus Figur 4 besitzt eine Ventilmembran 10 gemäß den Figuren 1A bis 2B. Im Gegensatz zu der in Figur 3 dargestellten Ausführungsform erfolgt hier die Fixierung über ein metallisches Geflecht 24.

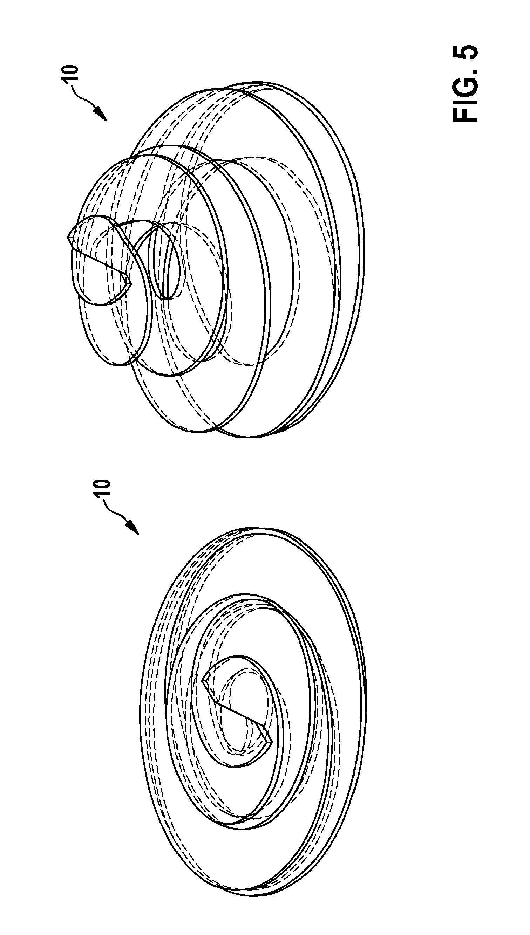

Der Figur 5 ist eine Ausführungsform der Ventilmembran 10 zu entnehmen, bei der 2 ineinander gewundene Spiralbänder so aufeinander im geschlossenen Zustand des Ventils zum Liegen kommen, dass die gesamte Grundfläche der Ventilmembran undurchlässig ist. Im geöffneten Zustand nehmen beide Spiralbänder wieder die Kontur einer kegelförmigen Raumspirale ein.

## Patentansprüche

1. Ventil für eine Herzklappenprothese (20) mit einer Ventilmembran (10) aus wenigstens einem Spiralband, das im geschlossenen Zustand der Ventilmembran (10) die Form einer archimedischen Spirale einnimmt, wobei sich die äußeren Randbereiche des Spiralbandes (12) mit einen inneren Randbereich (14) des Spiralbandes (12) einer vorhergehenden Windung der Spirale überlappen.

2. Ventil nach Anspruch 1, bei dem die Ventilmembran (10) im geöffneten Zustand die Form einer konischen Spirale einnimmt.

3. Ventil nach Anspruch 1, bei dem die Spirale zwischen 2 bis 12 Windungen aufweist.

4. Ventil nach Anspruch 1, bei dem ein inneres Ende des Spiralbandes (12) als kreisförmige Scheibe ausgebildet ist.

5. Ventil nach Anspruch 1, bei dem die Membran aus einem metallischen Werkstoff besteht.

6. Ventil nach Anspruch 1, bei dem das Spiralband (12) eine Dicke im Bereich von 500 bis 1.000 Mikrometer aufweist.

7. Herzklappenprothese (20) mit einem Ventil nach einem der Ansprüche 1 bis 6.
